# EUROPEAN PATENT APPLICATION

(11) **EP 4 194 439 A1**
(43) Date of publication of application: **14.06.2023**
(21) Application number: 21853682.9
(22) Date of filing: 05.08.2021
(51) Int. Cl.: C07C 335/16, G01N 21/78, G01N 31/22, G01N 33/14, C07C 59/01

(54) **NEW THIOUREA-BASED COMPOUNDS, PRODUCTION METHOD AND USE THEREOF TO DETECT GHB IN BEVERAGES**

(30) Priority: 06.08.2020 ES 202030841
(71) Applicant: Universitat de València, 46010 Valencia (ES)
(72) Inventor: RODRIGUEZ NUÉVALOS, Silvia, 46010 VALENCIA (ES); COSTERO NIETO, Ana María, 46010 VALENCIA (ES); PARRA ÁLVAREZ, Margarita, 46010 VALENCIA (ES); GIL GRAU, Salvador, 46010 VALENCIA (ES); GAVIÑA COSTERO, Pablo, 46010 VALENCIA (ES); HERNANDEZ CONTRERAS, Jordi, 46010 VALENCIA (ES)
(74) Representative: Ponti & Partners, S.L.P
(86) International application number: PCT/ES2021/070596
(87) International publication number: WO 2022/029358

(57) **Abstract**

The present invention relates to compound 1 or 2 or the composition comprising it. The present invention also relates to a process for obtaining compound 1 or 2, as well as to the use thereof for detecting GHB in beverages. An equipment or kit for use in detecting GHB in beverages is also included.

## Description

### Field of the invention

The present invention relates to new thiourea-based compounds, to a process for obtaining them and to their use in the detection of γ-hydroxybutyric acid (GHB) in beverages.

### Background

γ-hydroxy butyric acid (GHB) is one of the drugs used in chemical submission. It is an odorless, colorless, slightly salty compound sometimes used for recreational purposes. However, this compound, when supplied without knowledge of the victim, has been used on numerous occasions in order to commit an offense. In many of them, it is used to void the will of the victims and carry out sexual assault. In general, the intake of the drug by the victim occurs through a beverage that has been contaminated with the product without noticing the product. Residence time in the body is 3-6 hours and its metabolites are excreted rapidly, so that it is very difficult to detect their presence in the body after the assault.

Four references addressing this subject have been found in the scientific literature (J. Forensic. Sci. 2004, 49, p. 379-387, Chem. Commun. 2013, 49, p. 6170-6172, Chem. Commun. 2014, 50, p. 2904-2906, J. Mat. Chem. B 2017, 8, p. 2736-2742).

The first case (J. Forensic. Sci. 2004, 49, p. 379-387) relates to an enzymatic reaction while the other three reviews are referring to chemical sensors.

Chem. Commun. 2014, 50, 2904-2906 describes the use of Compound A as chemosensor for detecting GHB. The interaction with the analyte was based on a hydrogen bonding interaction between the phenol group on the sensor and the carboxylate unit on GHB.

In addition, the same authors also described in Chem. Commun. 2013, 49, 6170- 6172 the use of Compound B for detecting the corresponding lactone (GBL), but again, the interaction was related to the formation of a hydrogen bond. However, in any case, soft drinks alone or mixed with alcoholic beverages were not tested as interferents.

On the other hand, J. Mat. Chem. B 2017, 8, 2736-2742 describes the preparation of a complex of Ir (C) capable of detecting GHB. The complex showed luminescence quenching in the presence of analyte that was observed using UV light.

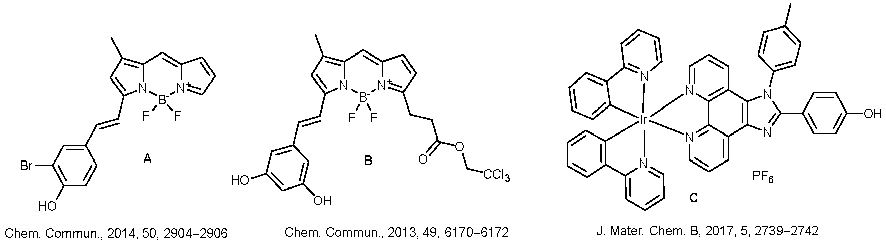

By following a different approach, Bravo D. T.; Harris, D. O., Parsons S. M. J. Forensic. Sci. 2004, 49, p. 379-387 described the use of γ-hydroxybutyrate dehydrogenase (GHB-DH) to detect GHB. Enzymatic oxidation of GHB by NAD⁺ is combined with reduction of a dye showing a colour change. Ethanol is an interferent that can be avoided by drying the sample to be studied.

The present inventors have been able to develop a reliable and easy-to-use procedure and device that enables visual determination of whether a beverage, whether alcoholic, non-alcoholic, or combined, has been contaminated with GHB prior to ingestion. Additionally, the ready-to-use device only needs a drop of the beverage under analysis so that in case the response is negative, it can be ingested substantially entirely without any problems.

### Summary of the Invention

In a first aspect, the present invention relates to a compound 1 or 2 according to claim 1.

In a second aspect, the present invention also relates to a composition comprising a compound 1 or 2 according to the first aspect.

In a third aspect, the present invention also relates to a process for obtaining a compound 1 or 2 according to the first aspect.

In a fourth aspect, the present invention relates to the use of a compound 1 or 2 according to the first aspect or a composition according to the second aspect for the detection of GHB in beverages.

In a fifth aspect, the present invention relates to a procedure for detecting GHB in beverages.

In a sixth aspect, the present invention relates to equipment or kit for use in detecting GHB in beverages.

### Brief description of the drawings

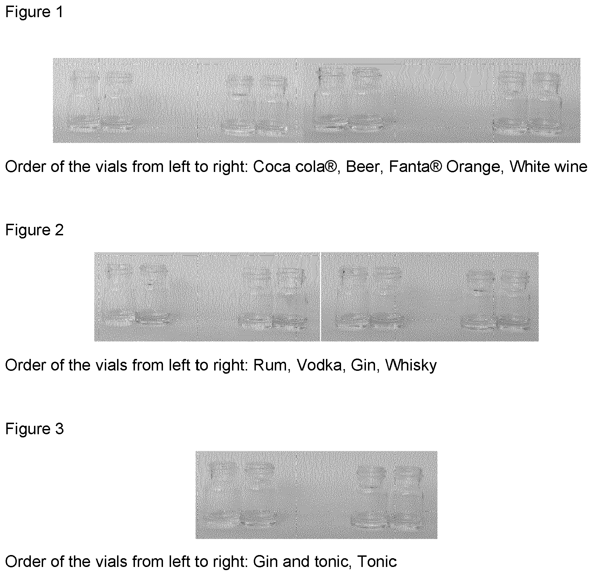

Figures 1 to 3 show the results of the real assay with different beverages for compound 2 (Figure 1 for Coca Cola^{®}, Beer, Fanta^{®} Orange, White Wine; Figure 2 for Rum, Vodka, Gin, Whisky; Figure 3 for gin and tonic, and tonic) where colour change is observed for contaminated beverages. The GHB in the beverage is at a concentration of 24 mM. After treatment with the base and mixing with dissolution of compound 1 or 2, it is 0.36 mM.

### Detailed description of the invention

In a first aspect, the present invention relates to a compound of formula 1 or 2:

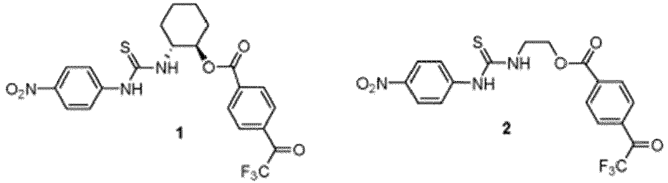

In a second aspect, the present invention relates to a composition comprising compound 1 or 2 as defined above. In a preferred embodiment, said composition comprises compound 1 or 2 as defined above and an organic solvent. As the organic solvent, but not limited thereto, DMF (dimethylformamide) and DMSO (dimethylsulfoxide) may be used, although DMSO is preferably utilized.

In a third aspect, the present invention relates to a process for obtaining compound 1 or 2, comprising the following reaction scheme:

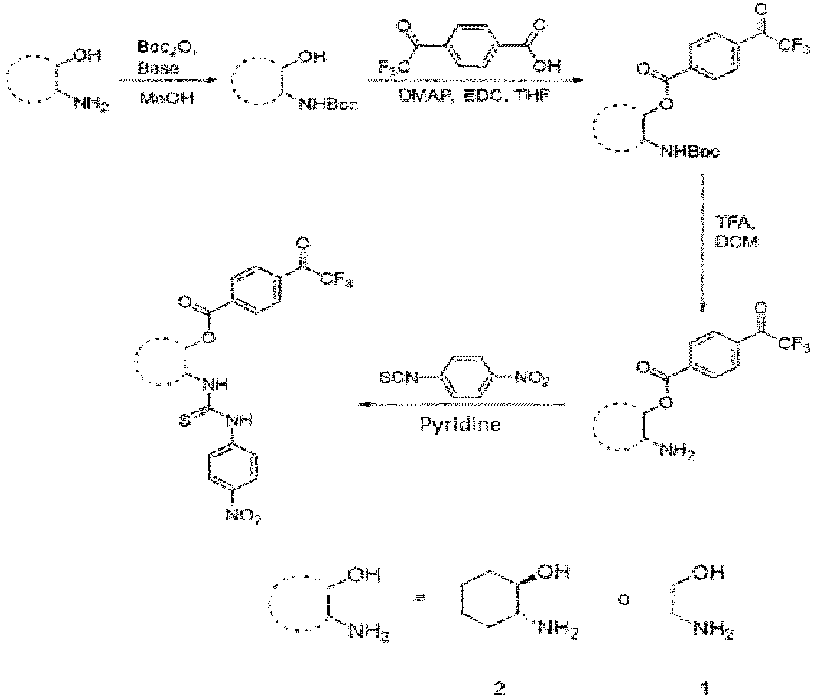

The reaction conditions are not particularly relevant, as it may be carried out at room P and T.

In a fourth aspect, the present invention relates to the use of a composition, as defined above, or a compound 1 or 2, as defined above, in the detection of γ-hydroxybutyric acid (GHB) or a salt thereof in beverages. Such salts may include, but are not limited to, salts with alkali or alkaline earth metals, preferably sodium. In the present invention, when reference is made to beverages, as indicated below, it will be understood as alcoholic beverages, non-alcoholic beverages or mixtures thereof. Where reference is made to alcoholic beverages, alcoholic distillates such as, for example, whisky or gin, as well as alcoholic beverages that are not distilled, such as wine and beer, are included.

In a fifth aspect, the present invention relates to a process for detecting GHB or a salt thereof in beverages, comprising:
a) taking a drop of the beverage to be analyzed and dissolving it in an aqueous solution of weak base;
b) adding an aliquot of the solution obtained in step a) to the composition, as defined above, or alternatively to compound 1 or 2, as defined above;
c) observing the reaction produced; and
d) qualitatively determining the presence of GHB in the beverage to be analyzed according to the observation of step c).

It should be noted that in step b) the composition of compound 1 or 2 and the organic solvent may already be prepared or may be prepared at that point of time just before the addition of the aliquot of the solution obtained in step a).

In a preferred embodiment, said weak base in step a) is sodium bicarbonate, although any weak base such as, for example, potassium, ammonium, calcium bicarbonate could be used. In another preferred embodiment, the concentration of the aqueous solution of weak base is from 0.4 mM to 1 mM, even more preferably 0.4 mM.

In another preferred embodiment, compound 1 or 2 is at a concentration between 50 µM and 100 µM, even more preferably 50 µM.

In step c) it should be observed if a reaction occurs between compounds 1 or 2, alone or in the composition, with the possible GHB component or a salt thereof present in the beverage to be analyzed. Whether compound 1 and compound 2 react positively with GHB or a salt thereof, a color change (from pale yellow to orange) will be observed. Thus, one can conclude in step d) about the presence or not of GHB in the beverage being analyzed.

In another alternative embodiment of the process, this may be slightly modified in the order of mixing, although the final result would be the same. In this regard, the alternative process comprises the steps of:
a) mixing an aqueous solution of a weak base as defined above with the organic solvent as defined above;
b) adding compound 1 or 2 as defined above;
c) immediately after step b), adding a drop of the beverage to be analyzed;
d) observing the reaction produced; and
e) qualitatively determining the presence of GHB in the beverage to be analyzed according to the observation of step d).

In another alternative embodiment of the process, the visual detection is performed on a strip of a non-woven fabric (NWF) with 50 to 100% polypropylene, preferably with 100% polypropylene. In this case, the process for detecting GHB or a salt thereof in beverages comprises the steps of:
a) introducing a strip of non-woven fabric containing at least 50% polypropylene into a beverage sample, typically between 3 and 5 seconds is enough;
b) introducing the beverage-impregnated strip of step a) into a container containing the composition of the invention, as defined above, or alternatively compound 1 or 2, as defined above, preferably the composition;
c) observing the reaction produced in the container; and
d) qualitatively determining the presence of GHB in the beverage to be analyzed according to the observation of step c).

Compound 1 or 2 is preferably at a concentration of 50 µM to 500 µM, preferably 50 µM to 100 µM.

The reaction is considered positive in GHB if in step c) a light yellow to intense orange change is observed with compounds 1 or 2.

This detection process with a fabric strip detects GHB in all types of pure beverages (alcoholic and non-alcoholic) and combined alcoholic and non-alcoholic beverages provided that it is at a concentration above 35 µM.

In a sixth aspect, the present invention relates to a kit or equipment for use in detecting GHB or a salt thereof in beverages comprising:
- a transparent container with opening and closure containing a composition as defined above, alternatively a compound 1 or 2, as defined above, optionally wherein said container containing compound 1 or 2 is accompanied by another container with the same characteristics but with organic solvent as defined above,
- a transparent container with opening and closure containing a weak base in the form of an aqueous solution;
- a tool for collecting a sample of the beverage to be analyzed.

In another embodiment, in the case of detecting GHB or a salt thereof in beverages by means of a strip of a non-woven fabric (NWF) with 50 to 100% polypropylene, the kit or equipment alternatively comprises:
- a strip of non-woven fabric (NWF) with 50 to 100% polypropylene;
- a transparent container with opening and closure containing a composition as defined above, alternatively a compound 1 or 2 as defined above, optionally wherein said container containing compound 1 or 2 is accompanied by another container with the same characteristics but with organic solvent as defined above, i.e. DMF (dimethylformamide) or DMSO (dimethylsulfoxide) are preferably used, more preferably DMSO.

Optionally, such a kit or equipment according to any of the preceding embodiments (whether based on strips for detection or not) further comprises instructions for use.

As noted above herein, the weak base used for the beverage sample in the kit without strip detection is preferably sodium bicarbonate, although any weak base such as potassium bicarbonate, ammonium bicarbonate, calcium bicarbonate could be used. In another preferred embodiment, the concentration of the aqueous solution of weak base is 0.4-1 mM, even more preferably 0.4 mM.

Additionally, as noted herein above, in the case of beverage detection for any of the embodiments of the kit (whether based on strips for detection or not), compound 1 or 2 is preferably present in the composition at a concentration between 50 µM and 500 µM, preferably from 50 µM to 150 µM, more preferably from 50 µM to 100 µM.

In a preferred embodiment, such transparent containers with opening and closure, according to any of the embodiments of the kit (whether based on strips for detection or not), are microtubes, graduated or not graduated, of polypropylene (e.g., Eppendorf^{™}).

In another preferred embodiment of the non-strip based kit, said tool for collecting the sample can be a dropper or a small spoon. In the case of the strip-based kit, said tool is not necessary.

In a more preferred embodiment, in the use, process, or kit or equipment, as defined in the foregoing aspects, the beverage is an alcoholic beverage. In another more preferred embodiment, said alcoholic beverage is admixed with a non-alcoholic beverage, preferably a soft drink or juice. In another more preferred embodiment, said beverage is only a non-alcoholic beverage, preferably a soft drink or a juice.

Preferably, said alcoholic beverage is selected from, by way of non-limiting example, gin, vodka, rum, whisky, white wine, vermouth, and beer.

Preferably, said soft drink or juice is selected from, by way of non-limiting example, tonic, orange soft drink, lemon soft drink, cola drink, tea drink and any fruit juice.

It should be noted that any combination of previous embodiments or instances or examples encompassed by the present invention is understood by one skilled in the art as being feasible in the context of the present invention.

The various particular instances or examples recited above, as well as the following examples, are provided for illustrative purposes only and are not intended to limit the scope of the present invention in any way.

### EXAMPLES

### Example 1. Synthesis of compound 1

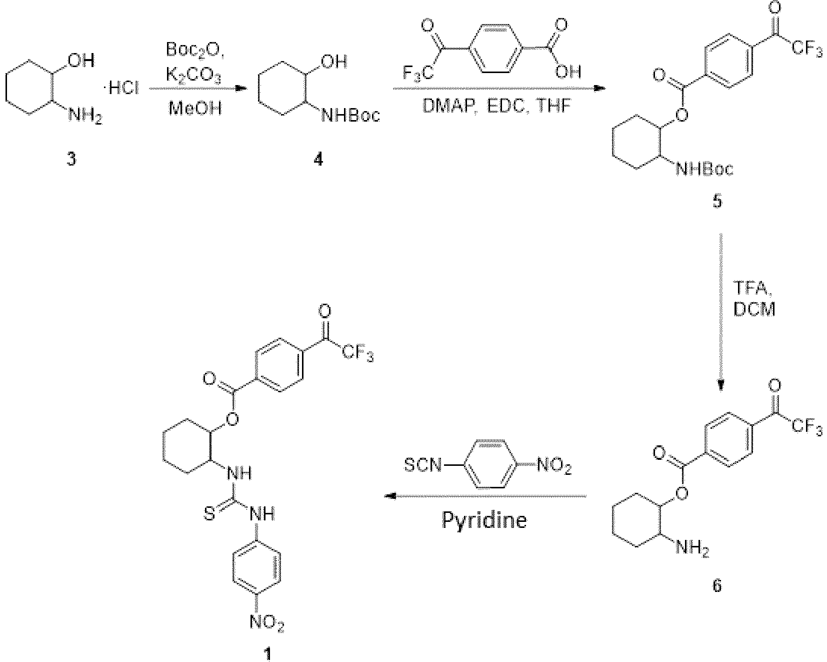

### Synthesis of Compound 4

In 50 mL of MeOH, 500 mg of **3** (3.30 mmol) and 842 mg of Na₂CO₃ (7.91 mmol) were stirred for 30 min and then 910 µL of Boc₂O (3.96 mmol) was added dropwise. Stirring was continued at room temperature until the next day. The MeOH was then removed under vacuum and about 30 mL of deionized water was added. It was extracted with AcOEt (3 × 20 mL), the organic layer was dried over anhydrous MgSO₄, filtered and the solvent removed under vacuum. 635 mg of compound 4 was obtained in 89% yield.

### Synthesis of Compound 5

365 mg of 4-(trifluoroacetyl)benzoic acid (1.67 mmol), 202 mg (1.67 mmol) of DMAP and 300 µL (1.67 mmol) of EDC were mixed in 10 mL of dry THF under inert atmosphere for 30 min. 300 mg of 4 (1.39 mmol, 0.139 M) was then dissolved in 10 mL of dry THF and added dropwise. The reaction was kept for 2 days at room temperature and under argon atmosphere. The solvent was then removed under vacuum, the resulting yellow oil was dissolved in 20 mL of AcOEt and the organic layer was washed with 1% HCl (2 × 10 mL), NaHCOs (sat) (2 × 10 mL) and NaCl (sat) (10 mL). The organic layer was dried with anhydrous MgSO₄, filtered, and the solvent removed under vacuum. 524 mg of compound **5** (91% yield) was isolated.

### Synthesis of Compound 6

In 5 mL of dry DCM, 201 mg of **5** (0.48 mmol) was dissolved with 1 mL of TFA (13.06 mmol) under inert atmosphere. The mixture was stirred for 15 min and the solvent removed under vacuum. The colorless oil was dissolved in 15 mL of AcOEt and the organic layer was washed with Na₂CO₃ (sat) (2 × 10 mL) and NaCl (sat) (10 mL), dried over anhydrous MgSO₄ and filtered. After evaporating the solvent, the compound **6** as a white powder (121 mg, 80% yield) was obtained.

### Synthesis of Compound 1

52 mg of 4-nitrophenyl isothiocyanate (0.29 mmol) was mixed with 110 mg of **6** (0.35 mmol) in 5 mL of pyridine under inert atmosphere for 2 days. The solvent was then removed and the resulting yellow oil was dissolved in 15 mL of AcOEt. The organic layer was washed with 1 M HCl (10 mL), NaHCOs (sat) (10 mL) and NaCl (sat) (10 mL), dried over anhydrous MgSO₄ and filtered. The solvent was removed under vacuum and the reaction crude was purified by chromatographic column using Hexane:AcOEt. AcOEt as eluent (from 7:3 to 6:4). 68 mg of compound 1 was obtained (47% yield).

### Example 2. Synthesis of compound 2

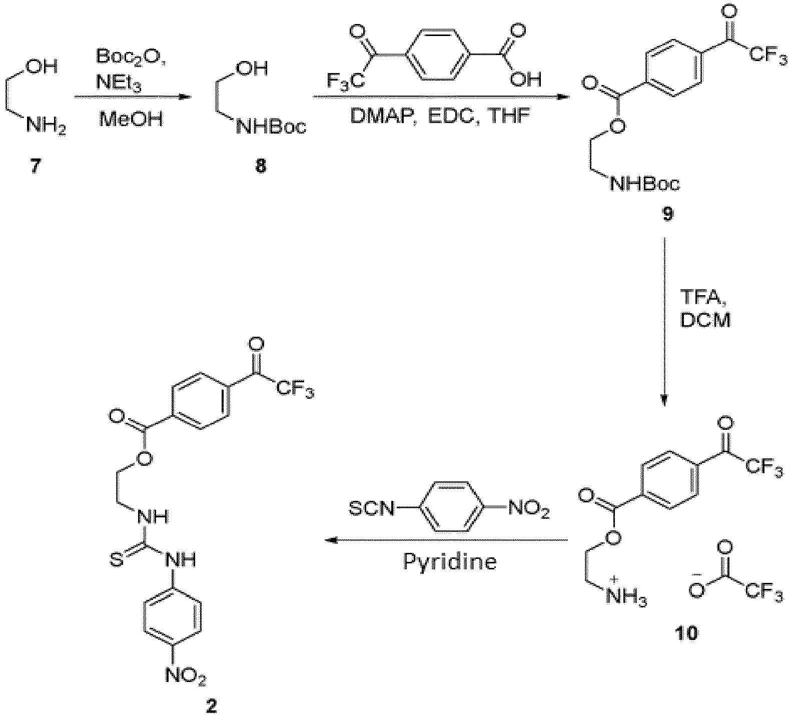

### Synthesis of Compound 8

In 10 mLof MeOH, 500 µL of 7 (8.31 mmol) and 1400 µL of Nets (9.97 mmol) were mixed and then 2300 µL of Boc₂O (9.97 mmol) was added dropwise. Stirring was kept at room temperature for 16 h. The MeOH was then removed under vacuum and the colorless oil dissolved in 30 mL of AcOEt. The organic layer was washed with deionized water (2 × 15 mL) and NaCl (sat) (15 mL), dried over anhydrous MgSO₄, filtered, and the solvent was removed under vacuum. 676 mg of compound 8 (50% yield) was obtained.

### Synthesis of Compound 9

807 mg of 4-(trifluoroacetyl)benzoic acid (3.70 mmol), 452 mg of DMAP (3.70 mmol) and 660 µL of EDC (3.70 mmol) were dissolved in 10 mL of dry THF under inert atmosphere. After 45 min stirring, 500 mg of **8** was dissolved in 10 mL of dry THF and added thereto. Stirring was kept at room temperature for 2 days. The solvent was then removed under vacuum and the resulting yellow oil was dissolved in 30 mL AcOEt. The organic layer was washed with 1% HCl (15 mL), NaHCOs (sat) (2 × 15 mL) and NaCl (sat) (15 mL), dried over anhydrous MgSO₄ and filtered. 1 g of compound **9** was obtained in 90% yield.

### Synthesis of Compound 10

In 4.5 mL of dry DCM, 1 g of 9 (2.77 mmol) and 1 mL of TFA (13.06 mmol) were mixed for 15 min under inert atmosphere. The solvent was then removed and compound 10 was isolated (616 mg, 60% yield).

### Synthesis of Compound 2

566 mg of **10** (2.17 mmol, 0.109 M) and 323 mg of 4-nitrophenyl isothiocyanate (1.81 mmol) were dissolved in 20 mL of pyridine under inert atmosphere. Stirring was kept at room temperature for 2 days. The solvent was then removed under vacuum and the resulting yellow oil dissolved in 30 mL of AcOEt. The organic layer was washed with 1 M HCl (15 mL), NaHCOs (sat) (2 × 15 mL) and NaCl (sat) (15 mL), dried over anhydrous MgSO₄, filtered and the solvent removed under vacuum. The crude reaction was purified by chromatography column using Hexane:AcOEt (from 7:3 to 6:4) as eluent. 400 g of compound **2** was isolated in 50% yield.

### Example 3. Assays on real samples

Firstly, the beverages were contaminated with GHB at a concentration of 24 mM. An aliquot of 100 µL was then taken and mixed with 100 µL of NaHCOs (1 mM or 0.4 mM). 30 µL of this solution were then taken and mixed with 50 µL of a solution of Compound **1** or **2** (1 mM in DMSO) and 920 µL of DMSO. The same process was followed for samples without GHB.

Results are seen in Figures 1-3 for different beverages (Figure 1 for Coca-cola^{®}, Beer, Fanta^{®} Orange, White Wine; Figure 2 for Rum, Vodka, Gin, Whisky; Figure 3 for gin and tonic, and tonic) for compound 2. Although not seen in the figures, the same results were obtained with compound 1.

### Example 4. GHB detection in beverages through a polypropylene strip

A strip of a 100% polypropylene non-woven fabric (NWF) of 0.5 × 3.0 cm was introduced into the beverage for three seconds and then brought to a vial containing a 125 µM concentration sensor solution in DMSO. Upon contact of the strip with the sensor, the color change from light yellow to intense orange occurred for both compounds 1 and 2 The system detects all types of combinations provided that the concentration is above 35 µM.

## Claims

1. A compound of formula 1 or 2:

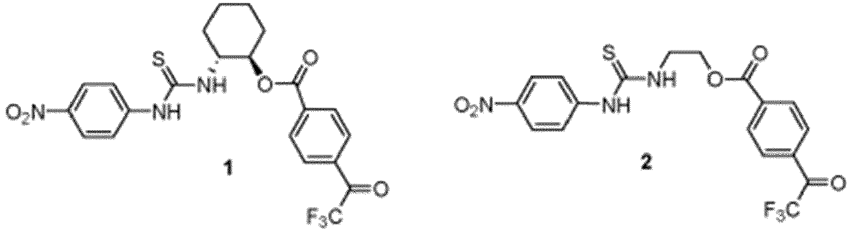

2. A composition comprising the compound according to Claim 1.

3. The composition according to Claim 2 comprising the compound according to Claim 1 and an organic solvent.

4. The composition according to Claim 2 consisting of the compound according to claim 1 and an organic solvent.

5. The composition according to claim 3 or 4, wherein the solvent is dimethylsulfoxide (DMSO).

6. A process for obtaining the compound according to Claim 1 with the following reaction scheme:

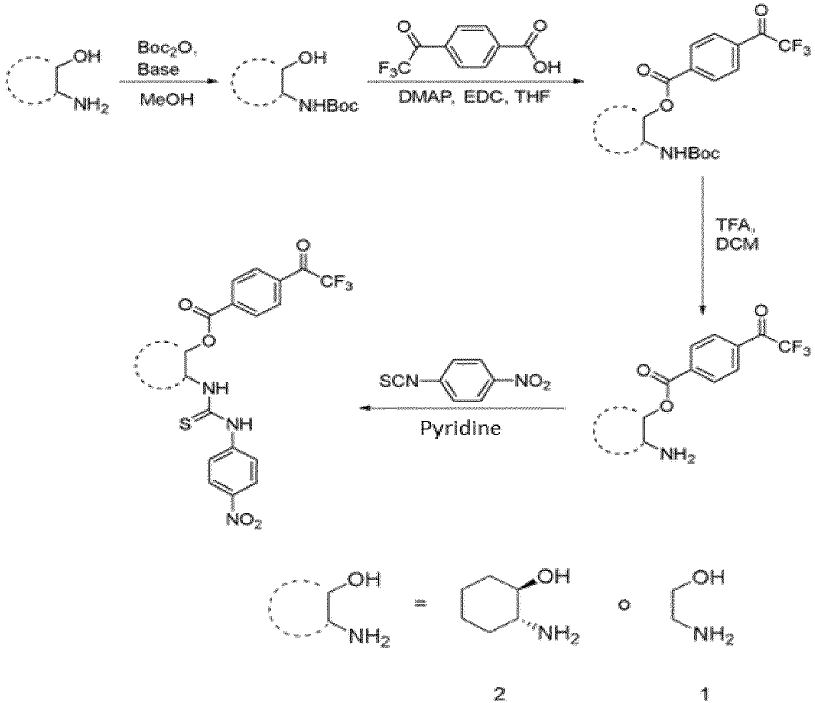

7. Use of a composition according to any of Claims 2 to 5, or compound according to Claim 1, in the detection of γ-hydroxybutyric acid (GHB) or a salt thereof in beverages.

8. The use according to Claim 7, wherein the beverage is an alcoholic beverage alone or mixed with a non-alcoholic beverage.

9. The use according to Claim 7, wherein the beverage is a non-alcoholic beverage alone.

10. The use according to Claim 8, wherein the alcoholic beverage is selected from gin, vodka, rum, whisky, white wine, vermouth and beer.

11. The use according to any of claims 8 to 10, wherein the non-alcoholic beverage is a soft drink or juice.

12. The use according to Claim 11, wherein the soft drink is selected from tonic, orange soft drink, lemon soft drink, tea soft drink and a cola soft drink.

13. A process for detecting GHB or a salt thereof in beverages, comprising:
a) taking a drop of the beverage to be analyzed and dissolving it in an aqueous solution of weak base;
b) adding an aliquot of the solution obtained in step a) to the composition, according to any of Claims 2 to 5, or to the compound 1 or 2, according to Claim 1;
c) observing the reaction produced; and
d) qualitatively determining the presence of GHB in the beverage to be analyzed according to the observation of step c).

14. A process for detecting GHB or a salt thereof in beverages, comprising:
a) mixing an aqueous solution of a weak base with an organic solvent;
b) adding compound 1 or 2 according to Claim 1 or 2;
c) immediately after step b), adding a drop of the beverage to be analyzed;
d) observing the reaction produced; and
e) qualitatively determining the presence of GHB in the beverage to be analyzed according to the observation of step d).

15. The process according to Claim 14, wherein the organic solvent is dimethylsulfoxide.

16. The process according to any of Claims 13 to 15, wherein the weak base is sodium, potassium, ammonium or calcium bicarbonate.

17. The process according to any of Claims 13 to 16, wherein the weak base is at a concentration between 0.4 mM and 1 mM.

18. A process for detecting GHB or a salt thereof in beverages comprising the steps of:
a) introducing a strip of non-woven fabric containing at least 50% polypropylene into a beverage sample;
b) introducing the beverage-impregnated strip of step a) into a container containing the composition according to any of Claims 2 to 5, or alternatively the compound 1 or 2 according to Claim 1;
c) observing the reaction produced in the container; and
d) qualitatively determining the presence of GHB in the beverage to be analyzed according to the observation of step c).

19. The process according to any one of Claims 13 to 18, wherein compound 1 or 2 is at a concentration in the composition between 50 µM and 500 µM.

20. The process according to any of Claims 13 to 19, wherein the beverage is an alcoholic beverage alone or mixed with a non-alcoholic beverage.

21. The process according to any of Claims 13 to 19, wherein the beverage is a non-alcoholic beverage alone.

22. The process according to Claim 20, wherein the alcoholic beverage is selected from gin, vodka, rum, whisky, white wine, vermouth and beer.

23. The process according to any of Claims 20 to 22, wherein the non-alcoholic beverage is a soft drink or juice.

24. The process according to Claim 23, wherein the soft drink is selected from tonic, orange soft drink, lemon soft drink, tea soft drink and a cola soft drink.

25. A kit or equipment for use in detecting GHB or a salt thereof in beverages comprising
- a transparent container with opening and closure containing a compound 1 or 2 according to Claim 1, or a composition according to any of Claims 2 to 5, optionally wherein said container containing compound 1 or 2 is accompanied by another container with the same characteristics but with organic solvent,
- a transparent container with opening and closure containing a weak base in the form of an aqueous solution;
- a tool for collecting a sample of the beverage to be analyzed.

26. A kit or equipment for use in detecting GHB or a salt thereof in beverages comprising
- a strip of non-woven fabric (NWF) with 50 to 100% polypropylene;
- a transparent container with opening and closure containing a composition according to any of Claims 2 to 5, alternatively a compound 1 or 2 according to Claim 1, optionally wherein said container containing compound 1 or 2 is accompanied by another container with the same characteristics but with organic solvent.

27. The kit or equipment according to claim 25 or 26 further comprising instructions for use.

28. The kit or equipment according to claim 25 or 27, wherein the weak base is sodium, potassium, ammonium or calcium bicarbonate.

29. The kit or equipment according to any one of claims 25, 27 or 28, wherein the weak base is at a concentration between 0.4 mM and 1 mM.

30. The kit or equipment according to any one of Claims 25 to 29, wherein compound 1 or 2 is at a concentration in the composition between 50 µM and 100 µM.

31. The kit or equipment according to any of claims 25 to 30, wherein the beverage is an alcoholic beverage alone or mixed with a non-alcoholic beverage.

32. The kit or equipment according to any of Claims 25 to 30, wherein the beverage is a non-alcoholic beverage alone.

33. The kit or equipment according to claim 31, wherein the alcoholic beverage is selected from gin, vodka, rum, whisky, white wine, vermouth and beer.

34. The kit or equipment according to any of claims 31 to 33, wherein the non-alcoholic beverage is a soft drink or juice.

35. The kit or equipment according to Claim 34, wherein the soft drink is selected from tonic, orange soft drink, lemon soft drink, tea soft drink and a cola soft drink.
